(19) Europäisches Patentamt
European Patent Office
Office européen des brevets

(11) EP 4 644 404 A1

(12) EUROPEAN PATENT APPLICATION
published in accordance with Art. 153(4) EPC

(43) Date of publication:
**05.11.2025 Bulletin 2025/45**

(21) Application number: **23912055.3**

(22) Date of filing: **25.12.2023**

(51) International Patent Classification (IPC):
***C07H 21/00*** *(2006.01)*

(52) Cooperative Patent Classification (CPC):
**C07H 21/00**

(86) International application number:
**PCT/JP2023/046424**

(87) International publication number:
**WO 2024/143276 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.12.2022 JP 2022208672**

(71) Applicant: **Sumitomo Chemical Company, Limited**
**Tokyo 103-6020 (JP)**

(72) Inventor: **TANAKA, Yuki**
**Oita-shi, Oita 870-0106 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) OLIGONUCLEOTIDE PRODUCTION METHOD

(57) The present invention provides a method for producing an oligonucleotide, comprising a step of decomposing branched products by reacting an n-polymerized oligonucleotide (where n is any integer of 2 or more) with water or an aqueous solution having a pH of 1 to 8, and an oligonucleotide wherein the content ratio of the branched products in the oligonucleotide is below a certain amount.

EP 4 644 404 A1

## Description

TECHNICAL FIELD

This application claims priority to and the benefit of Japanese Patent Application No. 2022-208672 filed December 26, 2022 according to the Paris Convention, the entire contents of which are incorporated herein by reference.
The present invention relates to a method for producing an oligonucleotide, comprising the selective decomposition of branched products, which are impurities in a synthetic oligonucleotide, under mild conditions.

BACKGROUND ART

In recent years, there has been increasing interest in the application of nucleic acid molecules in the medical field. For example, antisense nucleic acids, aptamers, ribozymes, and nucleic acids that induce RNA interference (RNAi) such as siRNA are mentioned, and these are called nucleic acid therapeutics.
An oligonucleotide can be synthesized using the phosphoramidite method (hereinafter referred to as the "amidite method"), and an oligonucleotide containing ribose is produced by deprotecting and removing a protecting group of a hydroxy group at a 2' position of ribose to obtain the desired oligonucleotide.
Branched products are known as the main impurities in the production of synthetic oligonucleotides (see Non-Patent Literature 1). Branched products are oligonucleotides bound to the base portion of a nucleotide through a phosphoramidate linkage. The formation of branched products leads to a problem where the yield and purity of the desired synthetic oligonucleotide decrease.
As a method for decomposing branched products, for example, a method of mixing triethylamine trihydrofluoride salt with the branched products to decompose them is known. (see Non-Patent Literature 2). Additionally, a method using 80% acetic acid water is also known for decomposing a phosphoramidate linkage (see Non-Patent Literature 3). However, these methods have severe reaction conditions, leading to the decomposition of the desired oligonucleotide as well. Therefore, there is a need for a method that selectively decomposes branched products under milder conditions.

CITATION LIST

NON-PATENT LITERATURE

Non-Patent Literature 1: Mass Spectrometry, Reviews 2021, 40, 75-109
Non-Patent Literature 2: Oligonucleotides 2006, 16, 181-185
Non-Patent Literature 3: J. Org. Chem., 1970, 35, 3800-3803


SUMMARY OF THE INVENTION

PROBLEMS TO BE SOLVED BY INVENTION

The present invention aims to provide a method for producing an oligonucleotide with reduced content of branched products, which comprises, in the method for producing the oligonucleotide, treating an oligonucleotide under mild conditions to decompose branched products as by-products.

MEANS TO SOLVE PROBLEMS

The present inventors have diligently conducted research to achieve the above-mentioned objective and found that by reacting an oligonucleotide with water or an aqueous solution having a pH of 1 to 8, selective decomposition of branched products proceeds, thereby improving the yield and purity of the resulting oligonucleotide. As a result, the present invention provides a method for producing an oligonucleotide, comprising a step of decomposing branched products by reacting an n-polymerized oligonucleotide (where n is any integer of 2 or more) with water or an aqueous solution having a pH of 1 to 8, and an oligonucleotide wherein the content ratio of the branched products in the oligonucleotide is below a certain amount.
The present invention encompasses the following embodiments but are not limited thereto.

[1] A method for producing an oligonucleotide, comprising a step of decomposing branched products by reacting an n-polymerized oligonucleotide (where n is any integer of 2 or more) with water or an aqueous solution having a pH of 1 to

8.

[2] The method for producing the oligonucleotide according to [1], wherein the step of decomposing the branched products comprises a reaction that selectively cleaves the phosphoramidate bonds of the branched products.

[3] The method for producing the oligonucleotide according to [1] or [2], wherein a crude oligonucleotide after solid-phase synthesis is used as a starting material.

[4] The method for producing the oligonucleotide according to any one of [1] to [3], wherein the step of decomposing the branched product comprises a step of reacting for 10 minutes or more by mixing the n-polymerized oligonucleotide (where n is any integer of 2 or more) with the water or the aqueous solution having the pH of 1 to 8.

[5] The method according to any one of [1] to [4], wherein the reaction temperature is 0 to 60°C.

[6] The method according to any one of [1] to [5], wherein the water or the aqueous solution having the pH of 1 to 8 is an aqueous solution containing acetic acid or an acetate.

[7] The method according to any one of [1] to [5], wherein the water or the aqueous solution having the pH of 1 to 8 is a Tris-HCl buffer having a pH of 7 to 8.

[8] The method according to any one of [1] to [5], wherein the water or the aqueous solution having the pH of 1 to 8 is water.

[9] The method according to any one of [1] to [8], wherein the n-polymerized oligonucleotide is an n-polymerized oligonucleotide containing a nucleotide having 2'-OMe.

[10] The method according to any one of [1] to [9], wherein the n-polymerized oligonucleotide is an n-polymerized oligonucleotide containing a nucleotide having 2'-OH.

[11] The method according to any one of [1] to [10], wherein the pH of the aqueous solution is 1 to 2 and the reaction temperature is 0 to 30°C.

[12] The method according to any one of [1] to [10], wherein the pH of the aqueous solution is 3 to 4 and the reaction temperature is 0 to 50°C.

[13] The method according to any one of [1] to [10], wherein the pH of the aqueous solution is 5 to 8 and the reaction temperature is 20 to 60°C.

[14] An oligonucleotide wherein the content ratio of the branched products is 15% or less relative to the full-length product (FLP).

[15] An oligonucleotide wherein the content ratio of the branched products is 5% or less relative to the FLP.

[16] An oligonucleotide with a chain length of 50 or more, wherein the content ratio of the branched products is 5% or less relative to the FLP.

[17] An oligonucleotide with a chain length of 100 or more, wherein the content ratio of the branched products is 5% or less relative to the FLP.

[18] The method for producing the oligonucleotide according to any one of [1] to [13], wherein the oligonucleotide is RNA.

[19] The oligonucleotide according to any one of [14] to [17], wherein the oligonucleotide is RNA.

Effect of Invention

The present invention provides a method for producing an oligonucleotide, characterized by the efficient proceeding of a selective decomposition reaction of branched products, which are reaction by-products, by reacting an oligonucleotide with water or an aqueous solution having a pH of 1 to 8. The production method of the present invention is expected to improve the yield and purity of the produced oligonucleotide.

BRIEF DESCRIPTION OF THE DRAWINGS

[Figure 1] Figure 1 is Scheme A, which shows a typical example of producing a nucleic acid oligomer represented by formula (5) from a nucleic acid oligomer represented by formula (1) using the phosphoramidite method.

MODE FOR CARRYING OUT THE INVENTION

According to one embodiment of the present invention, the present invention relates to a method for producing an oligonucleotide, comprising a step of decomposing branched products by reacting an n-polymerized oligonucleotide (where n is any integer of 2 or more) with water or an aqueous solution having a pH of 1 to 8.

According to one embodiment of the present invention, a representative reaction scheme for the decomposition reaction of the branched products in the present invention is shown below.

[Chemical Formula 1]

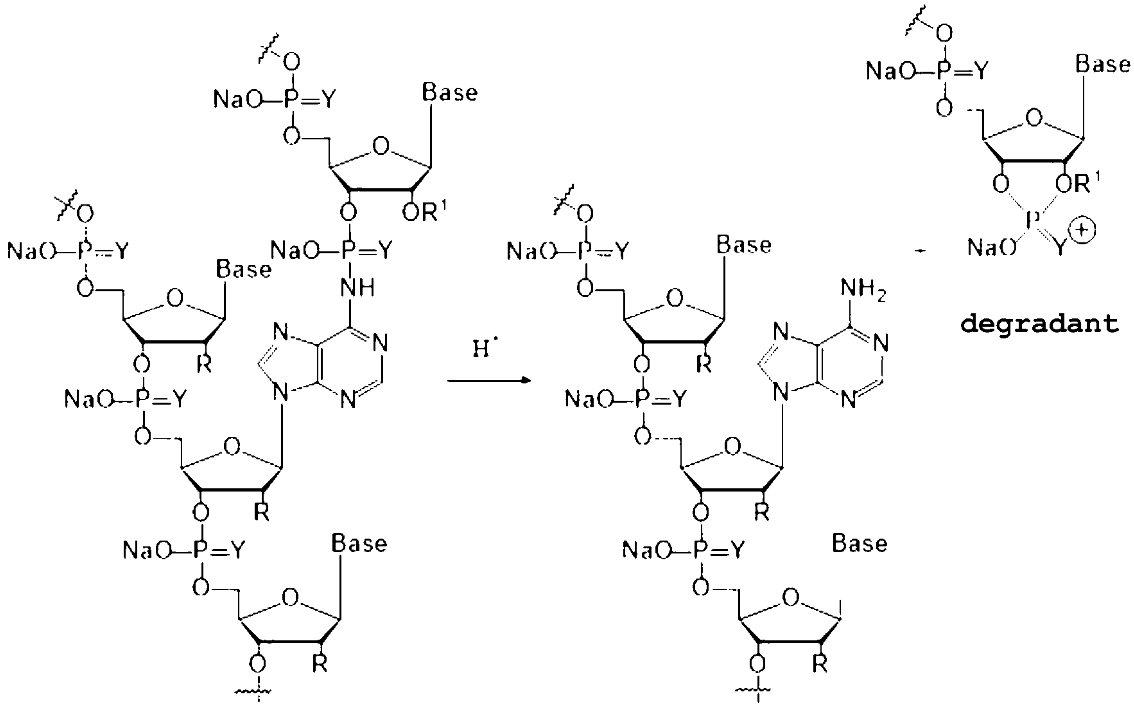


degradant

**Formula (A): branched products** FLP

In the formula, "Base", each of which is independently identical or different, represents a nucleobase, Y, each of which is independently identical or different, represents an oxygen atom or a sulfur atom, R, each of which is independently identical or different, represents a hydrogen atom, a fluorine atom, or an OQ group, Q, each of which is independently identical or different, represents a hydrogen atom, a methyl group, a 2-methoxyethyl group, a methylene group bonded to the 4' carbon atom of ribose, an ethylene group bonded to the 4' carbon atom of ribose, or an ethylidene group bonded to the 4' carbon atom of ribose, and $R^1$, each of which is independently identical or different, represents a hydrogen atom or an alkyl group, but is not limited thereto.

The term "branched products" used in this description refers to by-products in the production of a synthetic oligonucleotide. These are compounds in which an oligonucleotide is bound via a phosphoramidate bond at a base portion of a reactant nucleotide, and also comprises compounds where one or more additional nucleotides are polymerized to the nucleotide of the compound. The branched products are contained in a crude reaction product that comprises an oligonucleotide produced after the synthetic oligonucleotide production reaction. The term "crude oligonucleotide" or "crude oligonucleotide oligomer" used in this description refers to a mixture containing an oligonucleotide and branched products after the synthetic reaction.

The term "decomposing branched products" used in this description means cleaving the phosphoramidate bonds at the base portion of the aforementioned branched products. The term "selectively decomposing branched products" used in this description means selectively cleaving the phosphoramidate bonds at the base portion of the aforementioned branched products without cleaving phosphodiester bonds or phosphorothioate bonds within the desired target oligonucleotide.

As the term "water or an aqueous solution having a pH of 1 to 8" specifically refers to water or an aqueous solution of an acid or its salt with a pH of 1 to 8. Examples of the aqueous solution includes an aqueous solution comprising an organic acid or its salt, and an aqueous solution comprising an inorganic acid or its salt, specifically, include an aqueous solution comprising acetic acid or an acetate (e.g., sodium acetate, ammonium acetate, potassium acetate, calcium acetate), and Tris-HCl buffer, but are not limited to these examples.

Additionally, as water, UF water (ultrafiltration water) can be mentioned, but it is not limited to this. For an aqueous solution with a pH of 1 to less than 6.8, an aqueous solution of acetic acid or an acetate can be mentioned. UF water with a pH of 6.8 can be mentioned. For an aqueous solution with a pH greater than 6.8 and up to 8, Tris-HCl buffer can be mentioned.

In the present invention, the reaction conditions for "a step of decomposing branched products by reacting an n-polymerized oligonucleotide (where n is any integer of 2 or more) with water or an aqueous solution having a pH of 1 to 8"

require mixing the n-polymerized oligonucleotide (where n is any integer of 2 or more) with the water or the aqueous solution having the pH of 1 to 8 and allowing the reaction to proceed for a certain period of time or more. The reaction time may vary depending on the oligonucleotide reactant used, the water or the aqueous solution with the pH of 1 to 8, the reaction temperature, etc., but as long as the conditions under which the decomposition of the branched products can be sufficiently achieved, it is not particularly limited. For example, a period of 10 minutes or more is preferred. Specifically, it ranges from several tens of minutes to several weeks, including 10 minutes, 30 minutes, 1 hour, 2 hours, 3 hours, 6 hours, 12 hours, 24 hours, 48 hours (2 days), 72 hours (3 days), 1 week, several weeks, 1 month, and several months. Considering the stability of the obtained oligonucleotide in water or an aqueous solution with a pH of 1 to 8, a shorter reaction time is preferred, for example, at least 10 minutes or more, 30 minutes or more, or 60 minutes or more, while on the other hand, it is preferably no more than 1 week, no more than 72 hours, no more than 24 hours, and no more than 12 hours. Specific reaction times include, for example, 30 minutes to 1 week, 2 hours to 72 hours, and 6 hours to 24 hours, with a more preferred embodiment being 30 minutes to 24 hours.

The reaction temperature may vary depending on the oligonucleotide reactant used, the water or the aqueous solution with the pH of 1 to 8, the reaction time, etc., but as long as the conditions under which the decomposition of the branched products can be sufficiently achieved, it is not particularly limited. However, considering the stability of the resulting oligonucleotide, a lower reaction temperature is preferred. Specifically, temperatures of 0°C or higher, room temperature or higher (for example, 25°C or higher), 40°C or higher, and 50°C or higher are mentioned, while temperatures of 60°C or lower, 50°C or lower, and 40°C or lower are preferred. Specific reaction temperatures include, for example, 0°C to 60°C, 10 to 60°C, 20 to 60°C, 20 to 50°C, and 20 to 40°C.

As reaction conditions for the decomposition of the branched products, the relationship between pH and reaction temperature is not limited to these, but for example, conditions where the pH of the aqueous solution is 1 to 2 and the reaction temperature is 0 to 30°C, conditions where the pH of the aqueous solution is 3 to 4 and the reaction temperature is 0 to 50°C, and conditions where the pH of the aqueous solution is 5 to 8 and the reaction temperature is 20 to 60°C can be mentioned.

As reaction conditions for the decomposition of the branched products, although not limited to these, for example, conditions where the pH of the aqueous solution is 1 to 2, the reaction temperature is 0 to 30°C, and the reaction time is 10 minutes to 12 hours (for example, 30 minutes to 2 hours), conditions where the pH of the aqueous solution is 3 to 4, the reaction temperature is 0 to 50°C, and the reaction time is 6 hours to 48 hours (for example, 12 hours to 24 hours), and conditions where the pH of the aqueous solution is 5 to 8, the reaction temperature is 20 to 60°C, and the reaction time is 48 hours to several weeks (for example, 24 hours to 1 week) can be mentioned.

As reaction conditions for the decomposition of the branched products, generally, under more acidic conditions in pH, the decomposition reaction progresses faster, with lower reaction temperatures and shorter reaction times. On the other hand, under conditions closer to neutral pH, the decomposition reaction progresses more slowly, with higher reaction temperatures and longer reaction times.

In the term "branched products" used in this description, in the above formula (A), $R^1$ includes a hydrogen atom, an alkyl group (for example, a methyl group, an ethyl group), etc., but is not limited to these.

The term "n-polymerized oligonucleotide (where n is any integer of 2 or more)" used in this description refers to a polymerized oligonucleotide in which n (where n is any integer of 2 or more) nucleotides are polymerized through a phosphodiester bond or phosphorothioate bond between the 5' terminal and the 3' terminal of general nucleotides.

The specific structure of the n-polymerized oligonucleotide is not particularly limited, but for example, it includes an oligonucleotide containing a nucleotide having 2'-OMe and an oligonucleotide containing a nucleotide having 2'-OH.

In this description, "oligonucleotide" may also be referred to as "nucleic acid oligomer," and "nucleotide" may also be referred to as "nucleic acid molecule."

In this description, the crude oligonucleotide subjected to the decomposition reaction of the branched products may be a commonly known crude oligonucleotide before purification after liquid-phase synthesis, for example, a crude oligonucleotide with protected nucleobases, or a crude oligonucleotide before purification with deprotected nucleobases. Alternatively, it may be a crude oligonucleotide before purification after solid-phase synthesis, for example, a crude oligonucleotide with protected nucleobases, or a crude oligonucleotide before purification with deprotected nucleobases. A crude oligonucleotide after solid-phase synthesis is preferred.

In this description, the nucleoside (ribose and deoxyribose) contained within the nucleic acid molecule of the oligonucleotide may include both synthesized DNA and RNA, but preferably may include RNA.

The chain length of the oligonucleotide (N) is not particularly limited, but for example, it can be 2 chain length (mer) or more, 3 mer or more, 5 mer or more, 10 mer or more, 20 mer or more, 30 mer or more, 40 mer or more, 50 mer or more, 60 mer or more, 80 mer or more, 100 mer or more, 150 mer or more, 200 mer or more, 250 mer or more, 300 mer or more, 2 mer or more to 300 mer or less, 2 mer or more to 200 mer or less, 10 mer or more to 300 mer or less, 10 mer or more to 200 mer or less, 10 mer or more to 150 mer or less, 20 mer or more to 300 mer or less, 20 mer or more to 200 mer or less, 20 mer or more to 100 mer or less, 50 mer or more to 300 mer or less, 50 mer or more to 200 mer or less, 50 mer or more to 100 mer or less, 80 mer or more to 300 mer or less, 80 mer or more to 250 mer or less, 80 mer or more to 200 mer or less, 100 mer or more to

300 mer or less, 100 mer or more to 250 mer or less, and 100 mer or more to 200 mer or less are exemplified, but not limited to these.

The content ratio of the branched products within the oligonucleotide obtained by the selective decomposition step of the branched products of the present invention is 15% or less, 14% or less, 13% or less, 12% or less, 11% or less, 10% or less, 9% or less, 8% or less, 7% or less, 6% or less, 5% or less, 4% or less, 3% or less, 2% or less, or 1% or less relative to the full-length product (FLP), and preferably 5.0% or less, less than 5.0%, 4.5% or less, 4.1% or less, 4.0% or less, 3.5% or less, 3.4% or less, 3.0% or less, 2.5% or less, 2.2% or less, 2.0% or less, 1.5% or less, or 1.0% or less, but is not limited to these examples. The content ratio of the branched products is determined by analyzing a predetermined amount of a sample of crude oligonucleotide or crude oligonucleotide oligomer using high-performance liquid chromatography (HPLC).

Here, in the oligonucleotide, the content of the branched products relative to the full-length product (FLP) in the oligonucleotide, that is, the content of the branched products (%) when the content of the full-length product (FLP) in the oligonucleotide is set to 100%, is defined as the "branched products content ratio".

The analysis of the branched products by HPLC is typically performed using an oligonucleotide HPLC column (for example, Thermo Fisher's DNAPac™ PA200). As the mobile phase, for example, a gradient using a $CH_3CN$ solution containing Tris-HCl buffer and urea as mobile phase A, and a $CH_3CN$ solution containing $NaClO_4$, Tris-HCl buffer, and urea as mobile phase B is employed. The UV detection wavelength is typically 260 nm.

Next, a method for producing a nucleic acid molecule using the phosphoramidite method (amidite method) will be explained.

As a precursor having a phosphite triester bond, a nucleic acid compound represented by formula (4) is exemplified.

[Chemical Formula 2]

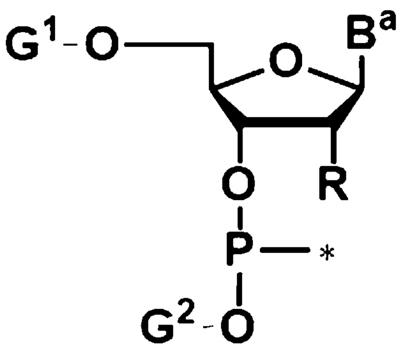

(4)

wherein,

$G^1$ represents a protecting group of a hydroxy group,
$G^2$, each of which is independently identical or different, represents a protecting group of a hydroxy group.
$B^a$, each of which is independently identical or different, represents a nucleobase which may be protected by a protecting group,
R, each of which is independently identical or different, represents a protected hydroxy group, hydrogen atom, a fluorine atom, a methoxy group, a 2-methoxyethyl group, an OQ' group, or an NQ' group,
Q', each of which is independently identical or different, represents an alkylene group or a carbonyl group, which are bonded to the carbon atom at the 4' position of the ribose.

In formula (4), when R represents an OQ' group or an NQ' group, and Q' represents an alkylene group or a carbonyl group, which are bonded to the carbon atom at the 4' position of the ribose, specific examples of such structures include LNA-1 to LNA-7 of the following formula (10).
Formula (10):

[Chemical Formula 3]

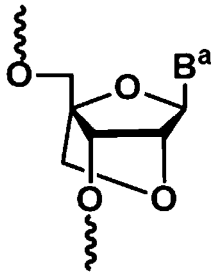
LNA-1

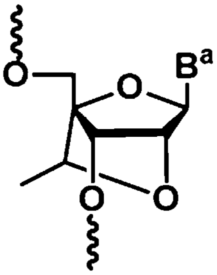
LNA-2

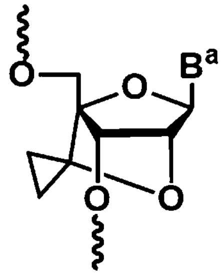
LNA-3

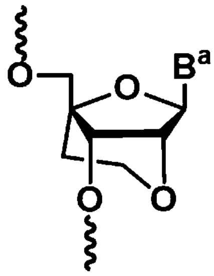
LNA-4

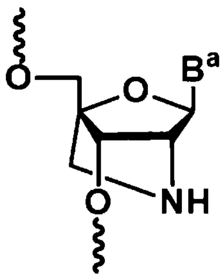
LNA-5

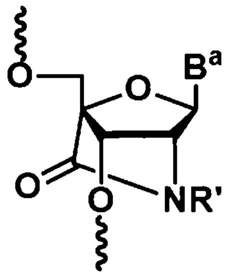
LNA-6

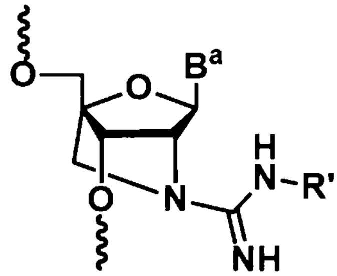
LNA-7

(10)

(wherein,

$B^a$ represents a nucleobase which may be protected,
R' represents a hydrogen atom or a methyl group.)

The nucleotide units contained in the nucleic acid molecule used in the present invention include DNA, RNA, 2'-O-Me, 2'-F, 2'-O-MOE (2'-O-methoxyethyl), UNA, morpholino nucleic acid, and the aforementioned LNA, but are not limited to these.

As a group represented by Z, which consists of a solid support and a connection group connecting the solid support with the oxygen atom of the 2' or 3' hydroxy group at the 3' terminal ribose of the nucleic acid oligomer (it is also referred to as an "oligonucleotide"), more specifically, a structure represented by the following formula (11) can be mentioned. More specifically, a structure represented by the following formula (11) can be mentioned.

[Chemical Formula 4]

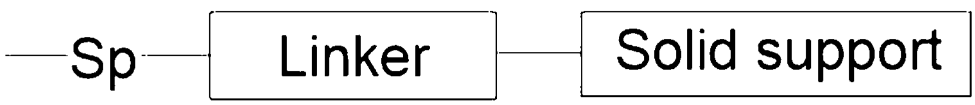


(11)

In formula (11), Sp represents a spacer.

The Spacer (Sp) is exemplified by a group having a structure represented by the following formula (12).

[Chemical Formula 5]

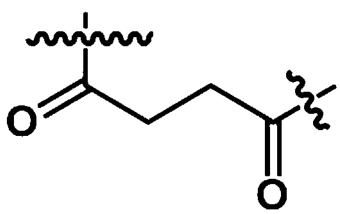

(12)

The linker may be, for example, a structure shown in the following formula (13), or a structure in which a hexamethyleneamino group portion in formula (13) is absent and an aminopropyl group is bonded to Si. Alternatively, the linker may be a structure shown in the following formula (14) .

[Chemical Formula 6]

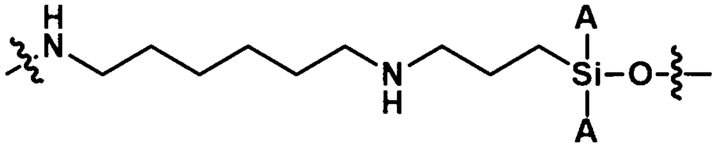

(13)

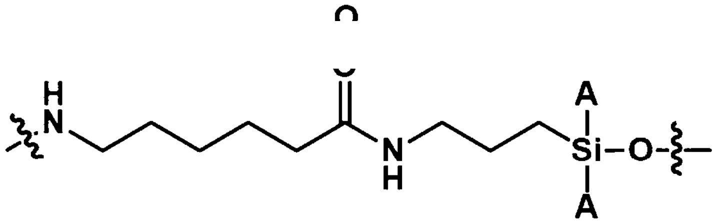

(14)

(wherein,
A may be a hydroxy group, an alkoxy group, or an alkyl group. Examples of the alkoxy group include a methoxy group and an ethoxy group. Examples of the alkyl group include a methyl group, an ethyl group, an isopropyl group, and an n-propyl group. Si indicates that it is bonded to the oxygen of a hydroxy group on the surface of a support.)

Examples of the solid support include an inorganic porous support and an organic resin support, and the others. Examples of the inorganic porous support include Controlled Pore Glass (CPG) and zeolite. Examples of the organic resin support include a support composed of polystyrene.

Various steps in the synthesis method of a nucleic acid molecule by the solid-phase synthesis method can be conducted under an atmospheric atmosphere, but it is preferable to conduct it under an inert gas (e.g., nitrogen, argon) atmosphere.

The method for synthesizing a nucleic acid molecule by the solid-phase synthesis method typically comprises the following steps.

(1) a step of deprotecting a hydroxy group at a 5' position of a nucleoside whose hydroxy group is protected and which is bonded to a solid support via a linker;
(2) a step of coupling reaction of the hydroxy group at the 5' position produced in the previous step with an amidite to obtain a phosphite triester compound;
(3) a step of oxidizing the phosphite triester produced in the previous step to convert it into a phosphate triester to produce an elongated nucleic acid molecule;
(4) a step of synthesizing a nucleic acid molecule on the solid support by repeating, any number of times, a series of reaction cycles composed of the steps (1) to (3), that is, the deprotecting step of the hydroxy group at the 5' position of the produced nucleic acid molecule, the coupling step of the hydroxy group at the 5' position with the amidite compound, and the oxidizing step of the produced phosphite triester;
(5) a step of subjecting the nucleic acid molecule on the solid support produced in the step (4) to a step of cleavage and deprotection to release it from the solid support to produce a nucleic acid molecule with the protecting group removed therefrom; and
(6) a step of deprotecting a protecting group of a hydroxy group at a 2' position of a ribose or at a 3' position of a 3' terminal, constituting a nucleic acid molecule.

However, in the method for synthesizing the nucleic acid molecule, a step of capping a hydroxy group at a 5' position where the coupling reaction with the amidite did not proceed may be included following the step (2) or (3), and a capping step may be added at any point during the series of reaction cycles constituting the step (4).

The step (5) is more specifically conducted in the order of the following reactions (5-1) and (5-2) for the nucleic acid molecule on the solid support produced in the step (4). Here, the reaction of step (5-1) may be optionally conducted, and the reaction of step (5-2) may be conducted using the method described in JP 4705716 B2. As a result, it is possible to produce a nucleic acid molecule with the protecting groups removed from the nucleic acid molecule released from the solid support, or a nucleic acid molecule with the 5' terminal hydroxy group protected.

(5-1) a reaction of deprotecting a protecting group of a hydroxy group at a 5' terminal of a nucleic acid molecule;

(5-2) a reaction of cleaving and releasing a nucleic acid molecule from a solid support, and a reaction of deprotecting protecting groups of nucleobases.

The step (6) is more specifically conducted by subjecting the nucleic acid molecule, which is obtained in the step (5), released from the solid support, and deprotected, to the deprotection reaction of the following step (6).
(6) a reaction of deprotecting the protecting group of the hydroxy group at the 2' position of the ribose or at the 3' position of the 3' terminal, constituting the nucleic acid molecule.
The scheme of the steps (1) to (6) is shown as Scheme A in Figure 1. The synthesis of a nucleic acid compound by the amidite method in the steps (1) to (5) can be conducted by repeating the deprotection step and the coupling step according to generally known methods (for example, the methods described in JP 5157168 B2 or JP 5554881 B2), thereby conducting the nucleic acid elongation reaction.
The following is an explanation of each step. Among the substituents in the chemical formulas in Scheme A, the definitions of $G^1$, $G^2$, $B^a$, and R are as described above. Additionally, the definitions of $G^3$, $G^4$, $G^5$, $B^c$, and R' are as described later. Additionally, in the chemical formulas in Scheme A, Y, each of which is independently identical or different, represents an oxygen atom or a sulfur atom,

X represents an R group or an OZ group, where Z is as described above,
W represents an OZ group when X represents an R group, where Z is as described above, or W represents an OV group when X represents an OZ group, where V represents a protecting group of a hydroxy group,
W may include groups derived from the W group (for example, residues cleaved from a solid support or deprotected groups),
X may include groups derived from the X group (for example, residues cleaved from a solid support or deprotected groups),
n represents an integer from 1 to 300, and
m represents an integer from 1 to 300.

As for $G^1$, it can be used without any particular limitation as long as it can function as a protecting group, and publicly known protecting groups used for amidite compounds can be widely used.
$G^1$ is preferably the following group.

[Chemical Formula 7]

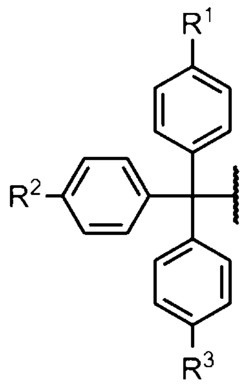

(wherein, $R^1$, $R^2$ and $R^3$ are each independently identical or different and represent hydrogen or an alkoxy group.)
One of $R^1$, $R^2$ and $R^3$ is hydrogen, and the remaining two thereof are identical or different (preferably identical) and represents preferably an alkoxy group, and as the alkoxy group, a methoxy group is particularly preferred.
As for $G^2$, it can be used without any particular limitation as long as it can function as a protecting group, and publicly known protecting groups used for amidite compounds can be widely used. Examples of $G^2$ include an alkyl group, an alkenyl group, an alkynyl group, a cycloalkyl group, a haloalkyl group, an aryl group, a heteroaryl group, an arylalkyl group, a cycloalkenyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri-alkylsilyloxyalkyl group, and the others, and these groups may be substituted with one or more electron-withdrawing groups.
$G^2$ is preferably an alkyl group substituted with an electron-withdrawing group. Examples of the electron-withdrawing group include a cyano group, a nitro group, an alkylsulfonyl group, a halogen atom, an arylsulfonyl group, a

trihalomethyl group, a trialkylamino group, and the others, and preferably a cyano group.

As for $G^2$, a 2-cyanoethyl group (represented by the following formula) is particularly preferred.

[Chemical Formula 8]

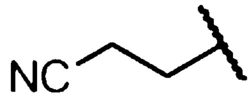

As for $G^3$, two $G^3$ may be combined with each other to form a cyclic structure. Preferably, both $G^3$ are an isopropyl group.

The alkyl group as the definitions of the above $R^1$, $R^2$, $R^3$, $G^2$, and $G^3$ may be a straight chain or a branched chain, and preferably an alkyl group containing 1 to 12 carbon atoms, and more preferably an alkyl group containing 1 to 6 carbon atoms. Specific examples of the alkyl group include methyl, ethyl, n-propyl, isopropyl, n-butyl, isobutyl, tert-butyl, n-pentyl, isopentyl, and n-hexyl. An alkyl group part which constitutes the alkoxy group in the definition for above substituents has the same definition as that described in the definition of the alkyl group described here.

In this description, a nucleobase refers to a group having a natural type or non-natural type nucleobase backbone. The nucleobase also includes modified forms in which the natural type or non-natural type nucleobase backbone is modified.

A nucleobase represented by $B^a$ which may be protected by a protecting group is not particularly limited. Examples of the nucleobase include adenine, cytosine, guanine, uracil, thymine, 5-methylcytosine, pseudouracil, 1-methylpseudouracil, and the others. In addition, the nucleobase may be substituted with substituent(s). Examples of the substituent include a halogen atom such as a fluoro group, a chloro group, a bromo group, and an iodo group, an acyl group such as an acetyl group, an alkyl group such as a methyl group and an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, a nitro group and the others, as well as combinations of two or more of these substituents.

When a nucleobase has an amino group outside the ring, a protecting group of the amino group is not particularly limited, and publicly known protecting groups used in nucleic acid chemistry can be used. Such protecting groups include, for example, a benzoyl group, a 4-methoxybenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, and a (dimethylamino)methylene group and the others, as well as combinations of two or more of these protecting groups.

As the nucleobase represented by $B^a$, more specifically, the following structures are exemplified.

[Chemical Formula 9]

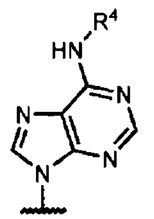

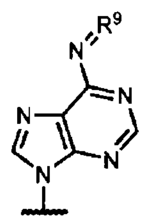

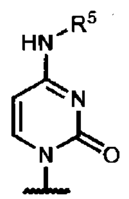

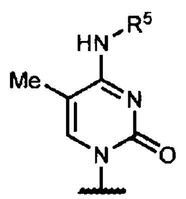

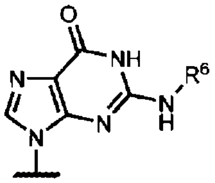 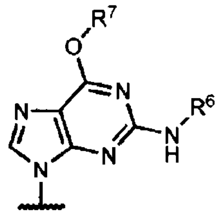 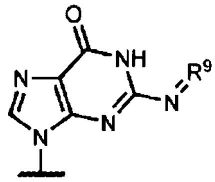 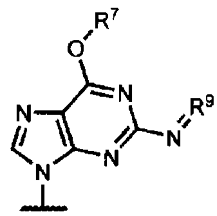

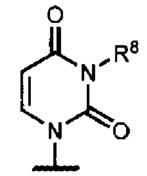 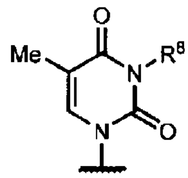 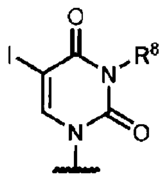 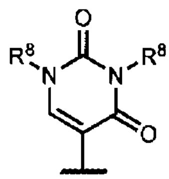

(wherein,

$R^4$ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
$R^5$ represents a hydrogen atom, an acetyl group, an isobutyryl group, or a benzoyl group,
$R^6$ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
$R^7$ represents a 2-cyanoethyl group,
$R^8$ represents a hydrogen atom, a methyl group, a benzoyl group, a 4-methoxybenzoyl group, or a 4-methylbenzoyl group, and
$R^9$ represents a dimethylaminomethylene group.)
It represents a group represented by any of the above.

In addition, in the method of the present invention, an amidite can be used in a free state or a salt state. Examples of the salt of the amidite include a base addition salt and an acid addition salt, but which are not particularly limited thereto. Specific examples of the base addition salt include salts with inorganic bases such as sodium salts, magnesium salts, potassium salts, calcium salts, aluminum salts and the others; salts with organic bases such as methylamine, ethylamine, ethanolamine and the others; salts with basic amino acids such as lysine, ornithine, arginine and the others; and ammonium salts. Specific examples of acid addition salts include mineral acids such as hydrochloric acid, hydrobromic acid, hydroiodic acid, sulfuric acid, nitric acid, phosphoric acid and the others; salts with organic acids such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethansulfonic acid, ethanesulfonic acid and the others; and salts with acidic amino acids such as aspartic acid, glutamic acid and the others. Examples of the amidite compounds encompass salts, hydrates, solvates, crystal polymorphs, and the others.

When R represents a protected hydroxy group, the protecting group can be any that can be used in the amidite method, examples include a 2'-tert-butyldimethylsilyl (TBDMS) group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group, a 2'-para-toluylsulfonylethoxymethyl (TEM) group, a 2'-EMM group (WO 2006/022323 A1), and those described in WO 2013/027843 A1 and WO 2019/208571 A1. Among these 2' protecting groups for ribonucleoside (RNA), a protecting group represented by formula (15) is exemplified as a preferred protecting group. More preferably, a protecting group represented by formula (16), having a cyano group as an electron-withdrawing group represented by $E_W$, is exemplified.

[Chemical Formula 10]

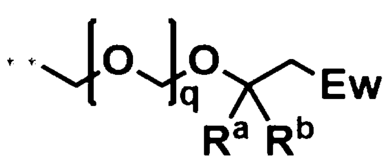 (15)

[Chemical Formula 11]

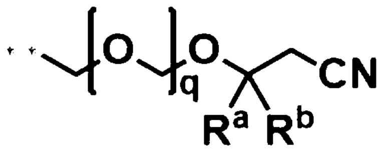

( 16 )

(wherein,

q represents an integer from 0 to 5,
$R^a$ and $R^b$ are each independently identical or different and represent a methyl group, an ethyl group, or a hydrogen atom,
the bond marked with ** is bonded to the oxygen of the protected hydroxy group, and
$E_W$ represents an electron-withdrawing group.)

The protecting group represented by formula (16) can be synthesized according to the descriptions in WO 2013/027843 A1 and WO 2019/208571 A1, for example. Amidite compounds having the protecting group can be used for the production of nucleic acid molecules.

In the elongation reaction of nucleic acids, the amidite of formula (3) described in Scheme A of Figure 1 is used.

(Nucleic Acid Elongation Reaction)

In this description, the "nucleic acid elongation reaction" refers to a reaction that elongates a nucleic acid molecule by sequentially connecting nucleotides via phosphodiester bonds or phosphorothioate bonds. The nucleic acid elongation reaction can be conducted according to the general amidite method (phosphoramidite method). The nucleic acid elongation reaction may be conducted using a nucleic acid automatic synthesizer that employs the amidite method.

The chain length of the nucleic acid oligomer can be, for example, 2 to 300 mer, 10 to 200 mer, or 15 to 150 mer.

The 5' deprotection step in the step (1) is a step of deprotecting a protecting group of a 5' hydroxy group at RNA chain terminal which is supported on a solid support. As a general protecting group, a 4,4'-dimethoxytrityl group (DMTr group), a 4-monomethoxytrityl group, and a 4,4',4"-trimethoxytrityl group are used. Deprotection can be conducted using an acid. Examples of the acid for deprotection include trifluoroacetic acid, dichloroacetic acid, trifluoromethanesulfonic acid, trichloroacetic acid, methanesulfonic acid, hydrochloric acid, acetic acid, p-toluenesulfonic acid, and the others.

The coupling step in the step (2) is a reaction where a nucleoside phosphoramidite represented by the following formula (3) described in Scheme A of Figure 1 is attached to the 5' hydroxy group at the oligonucleotide chain terminal deprotected in the above deprotection step. Note that the amidite compound represented by formula (3) is used as the amidite for nucleic acid elongation. Additionally, other usable amidites include 2'-OMe, 2'-F, a 2'-O-tert-butyldimethylsilyl group, a 2'-O-methoxyethyl group, 2'-H, 2'-fluoro-2'-deoxy-β-D-arabinofuranosyl, and the like. As the above nucleoside amidites, those where 5' hydroxy group is protected with a protecting group (for example, DMTr group) are used. The coupling step can be conducted by using an activator which activates the above-mentioned nucleoside amidite. Examples of the activator include 5-benzylthio-1H-tetrazole (BTT), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), 5-(bis-3,5-trifluoromethylphenyl)-1H-tetrazole, and the others.

The nucleoside amidite represented by formula (3) described in Scheme A of Figure 1 (hereinafter referred to as amidite) is as follows.

A compound represented by formula:

[Chemical Formula 12]

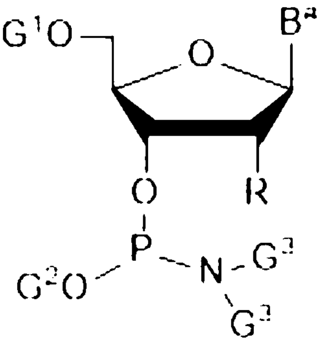

(3)

(wherein, $G^1$, $G^2$, $G^3$, $B^a$, and R are as described above.).

After the coupling step, as needed, an unreacted 5' hydroxy group may be capped. The capping can be conducted by using publicly known capping solutions such as an acetic anhydride-tetrahydrofuran solution, a phenoxyacetic anhydride/N-methylimidazole solution, and the others.

The oxidizing step in the step (3) is a step of converting a phosphite group which is formed in the above coupling step into a phosphate group or a thiophosphate group. This step is a reaction of converting a trivalent phosphorus into a pentavalent phosphorus using an oxidizing agent, which can be conducted by reacting an oxidizing agent with an oligonucleic acid derivative supported on a solid support.

When a phosphite group is converted into a phosphate group, as "oxidizing agent", for example, iodine can be used. The oxidizing agent can be prepared and used at a concentration of 0.005 to 2 M. Water can be used as the oxygen source for oxidation, and pyridine, N-methylimidazole (NMI), N-methylmorpholine, or triethylamine can be used as the base to proceed the reaction. In addition, as for solvent, it is not particularly limited as long as it does not participate in the reaction, but acetonitrile, tetrahydrofuran (THF), or a mixture of these in any ratio can be used. For example, iodine/-water/pyridine/acetonitrile, iodine/water/pyridine, iodine/water/pyridine/NMI, or iodine/water/pyridine/THF can be used. The reaction temperature is preferably 5°C to 50°C. The reaction time is usually suitable between 1 minute and 30 minutes. The amount of reagent used is preferably 1 to 100 mol per 1 mol of the compound supported on the solid support, more preferably 1 to 10 mol.

When a phosphite group is converted into a thiophosphate group, as "oxidizing agent", for example, sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethylidene)amino]-3H-1,2,4-dithiazoline-3-thione (DDTT), and phenylacetyl disulfide (PADS) can be used. The oxidizing agent can be diluted with an appropriate solvent to a concentration of 0.001 to 2 M for use. The solvent used for the reaction is not particularly limited as long as it does not participate in the reaction, but examples include dichloromethane, acetonitrile, pyridine, or a mixture of these in any ratio. The oxidizing step may be conducted after the capping operation, or conversely, the capping operation may be conducted after the oxidizing step, and this order is not limited.

In the step (5), a step of deprotecting a phosphate protecting group involves applying an amine compound to deprotect a protecting group in the phosphate portion after the synthesis of the nucleic acid with the desired sequence is completed. Examples of the amine compound include, for example, diethylamine and the others as described in JP 4705716 B2.

The protecting group of the 5' hydroxy group of the nucleoside incorporated in the last stage of the elongation may be used for the column purification with the 5' protecting group as a tag after the below-mentioned procedures of cleaving from a solid support and deprotecting of a protecting group, or alternatively, the protecting group of the 5' hydroxy group may be deprotected after the column purification.

In the step (5), the cleavage of the nucleic acid oligomer, which has been elongated to the desired chain length on the solid support, from the solid support is typically conducted by using concentrated aqueous ammonia as a cleaving agent.

Further, using ammonia or an amine compound or the others, for example, an oligonucleotide chain is collected by cleaving from a solid support. Examples of the amine compound include methylamine, ethylamine, isopropylamine, ethylenediamine, diethylamine, and the others.

In the step (6), the protecting group of the 2' or 3' hydroxy group of the ribose in the nucleic acid compound (6) cleaved from the solid support can be removed according to the methods described in WO 2006/022323 A1, WO 2013/027843 A1, or WO 2019/208571 A1, to obtain the deprotected nucleic acid oligomer (7).

A nucleotide and an amidite wherein an R group in formula (4) is a substitute other than a hydroxy group can be produced from nucleosides which are synthesized according to known methods described in JP 3745226 B2 and so on, or

WO 2001/053528 A1, JP 2014-221817 A1 or known methods referred to in these documents. Further, they can be produced by using a commercially available compound in line with the method described in the below Examples or methods with appropriate modifications to these methods.

Examples of the nucleic acid molecule which can be produced according to the production method of the present invention include those wherein a nucleoside contained in the nucleic acid molecule is RNA, DNA, RNA containing 2'-O-MOE, 2'-O-Me, or 2'-F, and LNA, which is not limited thereto. For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included. Preferably, the nucleic acid molecule produced by the method of the present invention is RNA.

By using the production method of the present invention, it is possible to produce an oligonucleotide with a reduced content of branched products as one embodiment.

Specific examples of such oligonucleotide include, but are not limited to, the following:

An oligonucleotide wherein the content ratio of the branched products within the oligonucleotide is 15% or less relative to the full-length product (FLP).

An oligonucleotide wherein the content ratio of the branched products within the oligonucleotide is 5.0% or less relative to the full-length product (FLP).

An oligonucleotide wherein the content ratio of the branched products within the oligonucleotide is 4.5% or less relative to the full-length product (FLP).

An oligonucleotide wherein the content ratio of the branched products within the oligonucleotide is 4.1% or less relative to the full-length product (FLP).

An oligonucleotide wherein the content ratio of the branched products within the oligonucleotide is 3.4% or less relative to the full-length product (FLP).

An oligonucleotide wherein the content ratio of the branched products within the oligonucleotide is 3.0% or less relative to the full-length product (FLP).

An oligonucleotide wherein the content ratio of the branched products within the oligonucleotide is 2.5% or less relative to the full-length product (FLP).

An oligonucleotide wherein the content ratio of the branched products within the oligonucleotide is 2.2% or less relative to the full-length product (FLP).

An oligonucleotide wherein the content ratio of the branched products within the oligonucleotide is 2.0% or less relative to the full-length product (FLP).

An oligonucleotide wherein the content ratio of the branched products within the oligonucleotide is 1.5% or less relative to the full-length product (FLP).

An oligonucleotide wherein the content ratio of the branched products within the oligonucleotide is 1.0% or less relative to the full-length product (FLP).

An oligonucleotide with a chain length of 100 mer or more, wherein the content ratio of the branched products within the oligonucleotide is 15% or less relative to the full-length product (FLP).

An oligonucleotide with a chain length of 100 mer or more, wherein the content ratio of the branched products within the oligonucleotide is 5.0% or less relative to the full-length product (FLP).

An oligonucleotide with a chain length of 100 mer or more, wherein the content ratio of the branched products within the oligonucleotide is 4.5% or less relative to the full-length product (FLP).

An oligonucleotide with a chain length of 100 mer or more, wherein the content ratio of the branched products within the oligonucleotide is 4.1% or less relative to the full-length product (FLP).

An oligonucleotide with a chain length of 100 mer or more, wherein the content ratio of the branched products within the oligonucleotide is 3.4% or less relative to the full-length product (FLP).

An oligonucleotide with a chain length of 100 mer or more, wherein the content ratio of the branched products within the oligonucleotide is 3.0% or less relative to the full-length product (FLP).

An oligonucleotide with a chain length of 100 mer or more, wherein the content ratio of the branched products within the oligonucleotide is 2.5% or less relative to the full-length product (FLP).

An oligonucleotide with a chain length of 100 mer or more, wherein the content ratio of the branched products within the oligonucleotide is 2.2% or less relative to the full-length product (FLP).

An oligonucleotide with a chain length of 50 mer or more, wherein the content ratio of the branched products within the oligonucleotide is 15% or less relative to the full-length product (FLP).

An oligonucleotide with a chain length of 50 mer or more, wherein the content ratio of the branched products within the oligonucleotide is 5.0% or less relative to the full-length product (FLP).

An oligonucleotide with a chain length of 50 mer or more, wherein the content ratio of the branched products within the oligonucleotide is 4.5% or less relative to the full-length product (FLP).

An oligonucleotide with a chain length of 50 mer or more, wherein the content ratio of the branched products within the oligonucleotide is 4.1% or less relative to the full-length product (FLP).

An oligonucleotide with a chain length of 50 mer or more, wherein the content ratio of the branched products within the oligonucleotide is 3.4% or less relative to the full-length product (FLP).

An oligonucleotide with a chain length of 50 mer or more, wherein the content ratio of the branched products within the oligonucleotide is 3.0% or less relative to the full-length product (FLP).

An oligonucleotide with a chain length of 50 mer or more, wherein the content ratio of the branched products within the oligonucleotide is 2.5% or less relative to the full-length product (FLP).

An oligonucleotide with a chain length of 50 mer or more, wherein the content ratio of the branched products within the oligonucleotide is 2.2% or less relative to the full-length product (FLP).

An oligonucleotide with a chain length of 50 mer to 200 mer, wherein the content ratio of the branched products within the oligonucleotide is 15% or less relative to the full-length product (FLP).

An oligonucleotide with a chain length of 50 mer to 200 mer, wherein the content ratio of the branched products within the oligonucleotide is 5.0% or less relative to the full-length product (FLP).

An oligonucleotide with a chain length of 50 mer to 200 mer, wherein the content ratio of the branched products within the oligonucleotide is 4.5% or less relative to the full-length product (FLP).

An oligonucleotide with a chain length of 50 mer to 200 mer, wherein the content ratio of the branched products within the oligonucleotide is 4.1% or less relative to the full-length product (FLP).

An oligonucleotide with a chain length of 50 mer to 200 mer, wherein the content ratio of the branched products within the oligonucleotide is 3.4% or less relative to the full-length product (FLP).

An oligonucleotide with a chain length of 50 mer to 200 mer, wherein the content ratio of the branched products within the oligonucleotide is 3.0% or less relative to the full-length product (FLP).

An oligonucleotide with a chain length of 50 mer to 200 mer, wherein the content ratio of the branched products within the oligonucleotide is 2.5% or less relative to the full-length product (FLP).

An oligonucleotide with a chain length of 50 mer to 200 mer, wherein the content ratio of the branched products within the oligonucleotide is 2.2% or less relative to the full-length product (FLP).

An oligonucleotide with a chain length of 50 mer to 300 mer, wherein the content ratio of the branched products within the oligonucleotide is 15% or less relative to the full-length product (FLP).

An oligonucleotide with a chain length of 50 mer to 300 mer, wherein the content ratio of the branched products within the oligonucleotide is 5.0% or less relative to the full-length product (FLP).

An oligonucleotide with a chain length of 50 mer to 300 mer, wherein the content ratio of the branched products within the oligonucleotide is 4.5% or less relative to the full-length product (FLP).

An oligonucleotide with a chain length of 50 mer to 300 mer, wherein the content ratio of the branched products within the oligonucleotide is 4.1% or less relative to the full-length product (FLP).

An oligonucleotide with a chain length of 50 mer to 300 mer, wherein the content ratio of the branched products within the oligonucleotide is 3.4% or less relative to the full-length product (FLP).

An oligonucleotide with a chain length of 50 mer to 300 mer, wherein the content ratio of the branched products within the oligonucleotide is 3.0% or less relative to the full-length product (FLP).

An oligonucleotide with a chain length of 50 mer to 300 mer, wherein the content ratio of the branched products within the oligonucleotide is 2.5% or less relative to the full-length product (FLP).

An oligonucleotide with a chain length of 50 mer to 300 mer, wherein the content ratio of the branched products within the oligonucleotide is 2.2% or less relative to the full-length product (FLP).

An oligonucleotide with a chain length of 100 mer to 300 mer, wherein the content ratio of the branched products within the oligonucleotide is 15% or less relative to the full-length product (FLP).

An oligonucleotide with a chain length of 100 mer to 300 mer, wherein the content ratio of the branched products within the oligonucleotide is 5.0% or less relative to the full-length product (FLP).

An oligonucleotide with a chain length of 100 mer to 300 mer, wherein the content ratio of the branched products within the oligonucleotide is 4.5% or less relative to the full-length product (FLP).

An oligonucleotide with a chain length of 100 mer to 300 mer, wherein the content ratio of the branched products within the oligonucleotide is 4.1% or less relative to the full-length product (FLP).

An oligonucleotide with a chain length of 100 mer to 300 mer, wherein the content ratio of the branched products within the oligonucleotide is 3.4% or less relative to the full-length product (FLP).

An oligonucleotide with a chain length of 100 mer to 300 mer, wherein the content ratio of the branched products within the oligonucleotide is 3.0% or less relative to the full-length product (FLP).

An oligonucleotide with a chain length of 100 mer to 300 mer, wherein the content ratio of the branched products within the oligonucleotide is 2.5% or less relative to the full-length product (FLP).

An oligonucleotide with a chain length of 100 mer to 300 mer, wherein the content ratio of the branched products within the oligonucleotide is 2.2% or less relative to the full-length product (FLP).

As typical examples of nucleic acid molecule which can be used in the production method of the present invention, the following examples are indicated in addition to examples described in working examples, which are not

limited thereto.

Hereinafter, in a description of a sequence, U represents uridine (ST.25 format), C represents cytidine, A represents adenosine, and G represents guanosine.

Nucleic acid molecules having the following sequences (A) and (B) as described in WO 2019/060442 A1 are exemplified.

Sequence (A): 5'-AUGGAAUmACUCUUGGUUmACdTdT-3' (conforms to ST.25 format) (5'-ATGGAATmACTCTTGGTTmACdTdT-3' (conforms to ST.26 format)) (Antisense) (Sequence No. 1) 21 mer

Sequence (B): 5'-GUmAACmCmAAGAGUmAUmUmCmCmAUmdTdT (conforms to ST.25 format) (5'-GTmAACmCmAAGAGTmATmTmCmCmATmdTdT-3' (conforms to ST.26 format)) (Sense) (Sequence No. 2) 21 mer

In the sequences (A) and (B), Um represents 2'-O-methyluridine (ST.25 format), Tm represents 2'-O-methyluridine (ST.26 format), Cm represents 2'-O-methylcytidine, and dT represents thymidine. As described herein, unless stated otherwise, the abbreviations in the sequence may be applied to both of the ST.25 format and the ST.26 format.

A nucleic acid molecule as described in Daniel O'Reilly et al., Nucleic Acids Research, 2019, Vol. 47, No.2, 546-558 (refer to p. 553) is exemplified. Typical examples thereof include a nucleic acid molecule having the following sequence (C).

Sequence (C): 5'-AGAGCCAGCCUUCUUAUUGUUUUAGAGCUAUGCUGU-3' (conforms to ST.25 format) (5'-AGAGCCAGCCTTCTTATTGTTTTAGAGCTATGCTGT-3' (conforms to ST.26 format)) (Sequence No. 3) 36 mer
A nucleic acid molecule having the following sequence (D) as described in Nucleic Acids Research, 2019, Vol. 47, No. 2: 547 is exemplified.
Sequence (D): 5'-ACAGCAUAGCAAGUUAAAAUAAGGCUAGUCCGUUAUCAACUUGAAAAAGUGGCACCGAGUCGGUGCU-3' (conforms to ST.25 format) ((5'-ACAGCATAGCAAGTTAAAATAAGGCTAGTCCGTTATCAACTTGAAAAAGTGGCACCGAG TCGGTGCT-3' (conforms to ST.26 format)) (Sequence No. 4) 67 mer

A nucleic acid molecule as described in JP 2015-523856 A1 page 173 is exemplified. Typical examples thereof include a nucleic acid molecule having the following sequence (E).
Sequence (E): 5'-GUUUUCCCUUUUCAAAGAAAUCUCCUGGGCACCUAUCUUCUUAGGUGCCCUCCCUUGUUUAAACCUGACCAGUUAACCGGCUGGUUAGGUUUUU-3' (conforms to ST.25 format) ((5'-GTTTTCCCTTTTCAAAGAAATCTCCTGGGCACCTATCTTCTTAGGTGCCCTCCCTTGTT TAAACCTGACCAGTTAACCGGCTGGTTAGGTTTT-3' (conforms to ST.26 format)) (Sequence No. 5) 94 mer

Nucleic acid molecules as described in JP 2017-537626 A1 are exemplified. Typical examples thereof include nucleic acid molecules having the following sequences (F), (G), (H) and (I).

Sequence (F): 5'-AGUCCUCAUCUCCCUCAAGCGUUUUAGAGCUAGUAAUAGCAAGUUAAAAUAAGGCUAGUCCGUUAUCAACUUGAAAAAGUGGCACCGAGUCGGUGCUUUU-3' (conforms to ST.25 format) ((5'-AGTCCTCATCTCCCTCAAGCGTTTTAGAGCTAGTAATAGCAAGTTAAAATAAGGCTAGT CCGTTATCAACTTGAAAAAGTGGCACCGAGTCGGTGCTTTT-3' (conforms to ST.26 format)) (Sequence No. 6) 100 mer

Sequence (G): 5'-GCAGAUGUAGUGUUUCCACAGUUUAAGAGCUAUGCUGGAAACAGCAUAGCAAGUUUAAAUAAGGCUAGUCCGUUAUCAACUUGAAAAAGUGGCACCGAGUCGGUGCUUUUUUU-3' (conforms to ST.25 format) ((5'-GCAGATGTAGTGTTTCCACAGTTTAAGAGCTATGCTGGAAACAGCATAGCAAGTTTAAA TAAGGCTAGTCCGTTATCAACTTGAAAAAGTGGCACCGAGTCGGTGCTTTTTTT-3' (conforms to ST.26 format)) (Sequence No. 7) 113 mer

Sequence (H): 5'-dAdGdTdCdCdTdCdAdTdCdTdCdCdCdTdCdAdAdGdCGUUUAAGAGCUAUGCUGGU AACAGCAUAGCAAGUUUAAAUAAGGCUAGUCCGUUAUCAACUUGAAAAAGUGGCACCGA GUCGGUGCUUUUUUU-3' (conforms to ST.25 format) ((5'-dAdGdTdCdCdTdCdAdTdCdTdCdCdCdTdCdAdAdGdCGTTTAAGAGCTATGCTGGT AACAGCATAGCAAGTTTAAATAAGGCTAGTCCGTTATCAACTTGAAAAAGTGGCACCGAGTCGGTGCTTTTTTT-3' (conforms to ST.26 format) (Sequence No. 8) 113 mer

In the sequence (H), dT represents thymidine, dC represents 2'-deoxycytidine, dA represents 2'-deoxyadenosine, and dG represents 2'-deoxyguanosine.
Sequence (I): 5'-AmsGmsUmsCCUCAUCUCCCUCAAGCGUUUAAGAGCUAUGCUGGUAACAGCAUAGCAAG UUUAAAUAAGGCUAGUCCGUUAUCAACUUGAAAAAGUGGCACCGAGUCGGUGCUUUUms UmsUmsU-3' (conforms to ST.25 format) ((5'-AmsGmsTmsCCTCATCTCCCTCAAGCGTTTAAGAGCTATGCTGGTAACAGCATAGCAAG TTTAAATAAGGCTAGTCCGTTATCAACTTGAAAAAGTGGCACCGAGTCGGTGCTTTTms TmsTmsT-3' (conforms to ST.26 format)) (Sequence No. 9) 113 mer

In the sequence (I), "Um" represents 2'-O-methyluridine (ST.25 format), "Tm" represents 2'-O-methyluridine (ST.26 format), "Am" represents 2'-O-methyladenosine, "Gm" represents 2'-O-methylguanosine, and "s" represents

phosphorothioate modification.

EXAMPLES

Hereinafter, the present invention is explained in more detail by working examples, but the present invention is not limited to these examples.

In this description, Me represents a methyl group. UF water refers to ultrafiltration water.

Measurement methods

First, various measurement methods used in the following tests are shown below.

(Measurement method 1: Measurement of the FLP ratio and the branched products ratio in the oligonucleotide)

The FLP ratio and the branched products ratio in the oligonucleotide were measured using HPLC. FLP stands for Full Length Product.

The HPLC measurement conditions are shown in Table 1 below.

[Table 1]

Table 1

| | |
|---|---|
| Column | DNAPac™ PA200 4 × 250mm |
| Flow rate | 1.0mL/min |
| Detection wavelength | 260nm |
| Mobile phase A | 25mM Tris-HCl buffer (pH=8.0), 10% $CH_3CN$, 6M Urea water |
| Mobile phase B | 500mM $NaClO_4$, 25mM Tris-HCl buffer (pH=8.0), 10% $CH_3CN$, 6M Urea water |
| Gradient | B conc (%) 20%(0min)-60%(60min)-90%(60.01min) -90%(65min)-20%(65.01min)-20% (80min) |
| Column temperature | 80°C |
| Injection volume | 10μL |

Solid-phase synthesis of oligonucleotides

Sequence (J): 5'-AmsUmsAmsACUCAAUUUGUAAAAAAGUUUUAGAGCUAGAAAUAGCAAGUUAAAAUAAG GCUAGUCCGUUAUCAACUUGAAAAAGUGGCACCGAGUCGGUGCUmsUmsUmsU-3' (conforms to ST.25 format) (5'-AmsCmsTmsCAATTTGTAAAAAAGTTTTAGAGCTAGAAATAGCAAGTTAAAATAAGGCT AGTCCGTTATCAACTTGAAAAAGTGGCACCGAGTCGGTGCTTTTmsTmsTmsT-3' (conforms to ST.26 format)) (Sequence No. 10) 100 mer

In the sequence (J), "A" is represented by the substructure delineated between the wavy lines in the following formula (A1). "C" is represented by the substructure delineated between the wavy lines in the following formula (A2). "G" is represented by the substructure delineated between the wavy lines in the following formula (A3). "U" is represented by the substructure delineated between the wavy lines in the following formula (A4). "Ums" is represented by the substructure delineated between the wavy lines in the following formula (A5). "Ams" is represented by the substructure delineated between the wavy lines in the following formula (A6). Additionally, "Ams" at the 5' terminal is represented by the upper substructure delineated between the wavy lines in the following formula (A7). Furthermore, "U" at the 3' terminal is represented by the lower substructure delineated between the wavy lines in the following formula (A8). However, the phosphate group in the structural formula may be in the form of a salt.

[Chemical Formula 13]

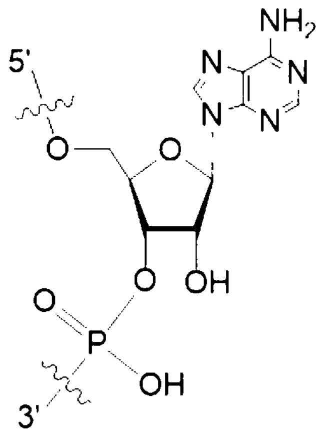

(A1)

[Chemical Formula 14]

[

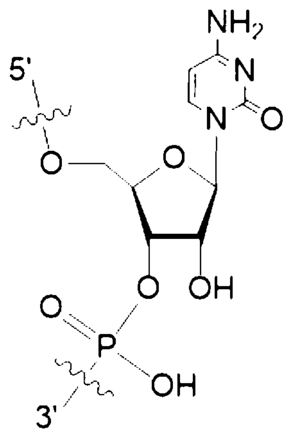

(A2)

[Chemical Formula 15]

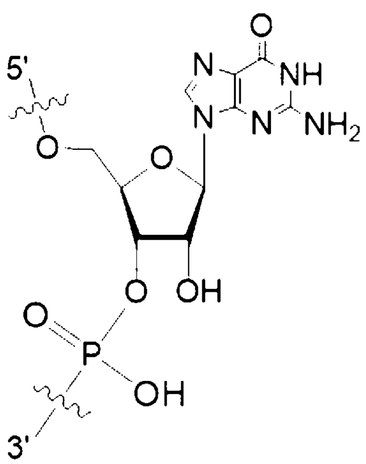

(A3)

[Chemical Formula 16]

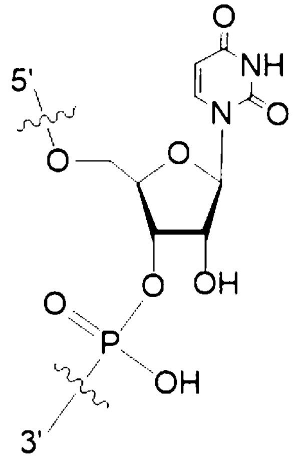

(A4)

[Chemical Formula 17]

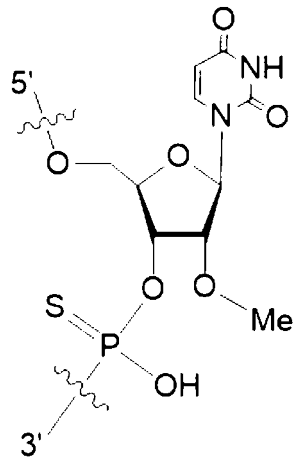

(A5)

[Chemical Formula 18]

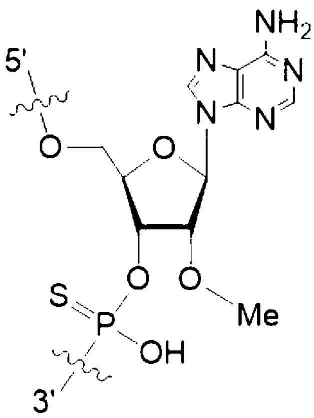

(A6)

[Chemical Formula 19]

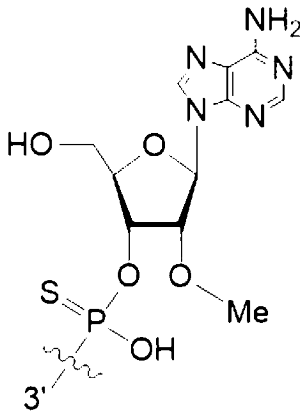

(A7)

[Chemical Formula 20]

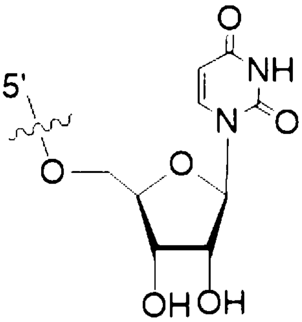

(A8)

Using Controlled Pore Glass (CPG) as the solid support and the AKTA oligopilot plus 100 (manufactured by GE Healthcare) as the nucleic acid synthesizer, an oligonucleotide consisting of the above sequence (J) was synthesized from the 3' terminal to the 5' terminal by the phosphoramidite solid-phase synthesis method. The synthesis was conducted on a 9.79 μmol scale. For the synthesis, adenosine PMM amidite (compound (A9)), cytidine PMM amidite (compound (A10)), guanosine PMM amidite (compound (A11)), and uridine PMM amidite (compound (A12)), described in WO 2019/208571 A1, as well as adenosine 2'-OMe amidite (compound (A13)) and uridine 2'-OMe amidite (compound (A14)) were used. A dichloroacetic acid toluene solution was used as the deblocking solution, 5-benzylmercapto-1H-tetrazole was used as the coupling agent, an iodine solution was used as the oxidizing agent, and a phenoxyacetic anhydride solution and an N-methylimidazole solution were used as the capping solutions. After the nucleic acid elongation was completed, a diethylamine solution was reacted with the nucleic acid on the support to selectively deprotect the cyanoethyl protecting group of the phosphate moiety. Here, PMM is an abbreviation for ((1-cyanopropan-2-yl)oxy)methoxy)methyl group.

[Chemical Formula 21]

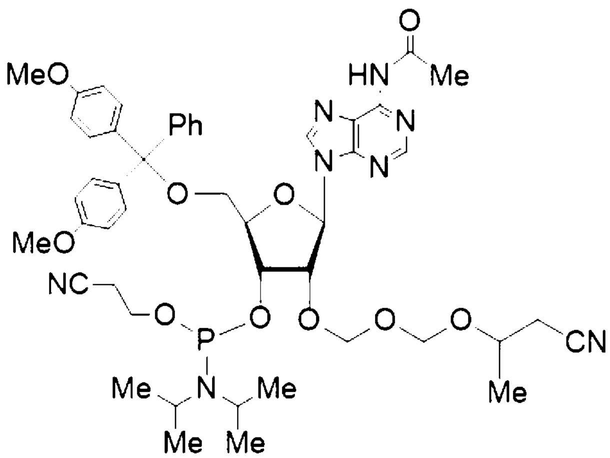

(A9)

[Chemical Formula 22]

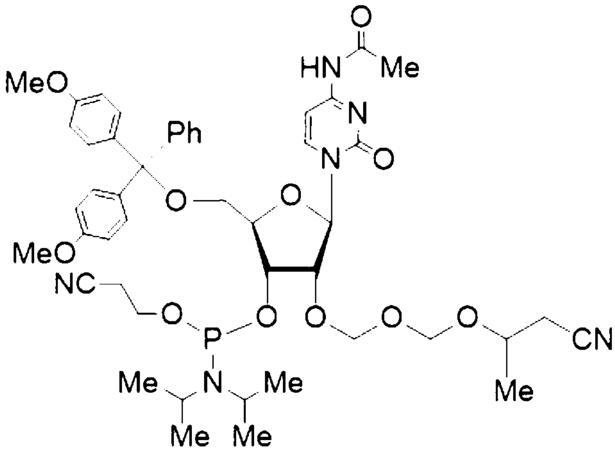

(A10)

[Chemical Formula 23]

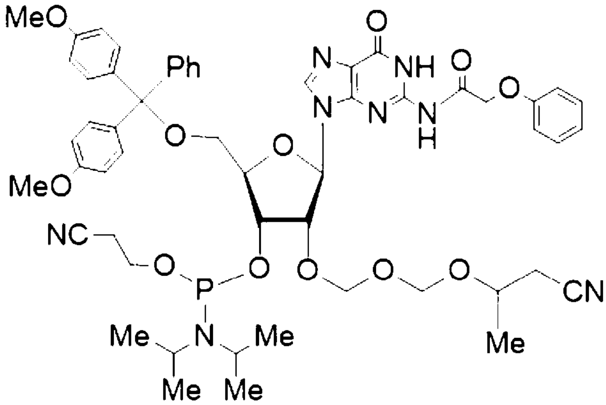

(A11)

[Chemical Formula 24]

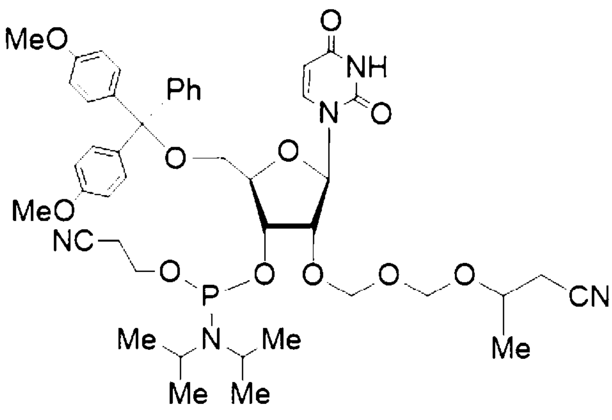

(A12)

[Chemical Formula 25]

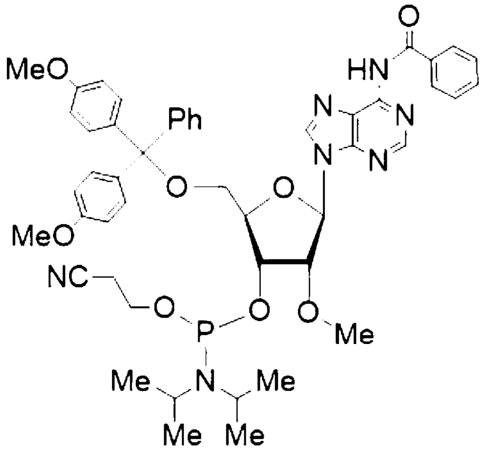

(A13)

[Chemical Formula 26]

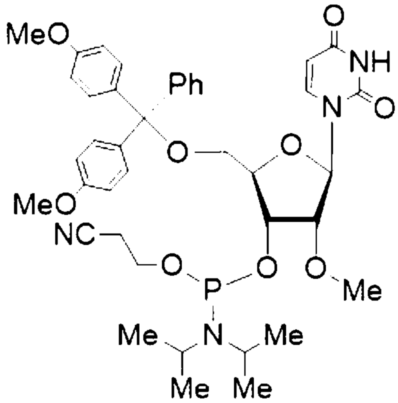

(A14)

Next, specific examples of the production of oligonucleotides (nucleic acid oligomers) manufactured by the method of the present invention are shown. Here, in the following examples, the oligonucleotide produced by the method of the present invention is an oligonucleotide having the sequence (J) represented by Sequence No. 10.

In addition, the uridine derivative described in the following examples and comparative examples refers to the compound represented by the following structural formula. The circle depicted in the structural formula schematically represents CPG.

[Chemical Formula 27]

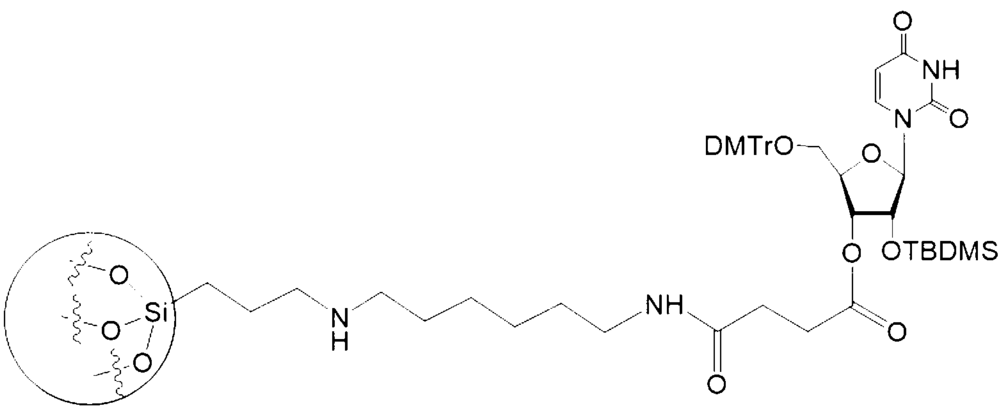

Reference Example 1

Using CPG loaded with 9.79 μmol of the uridine derivative, and amidites represented by formula (A9), formula (A10), formula (A11), formula (A12), formula (A13), or formula (A14), the solid-phase synthesis of sequence (J) was

conducted using the AKTA oligopilot plus 100. Subsequently, the CPG loaded with 5.01 μmol of the oligonucleotide was collected, and 2.85 mL of 28% aqueous ammonia and 0.95 mL of ethanol were added. The mixture was incubated at 40°C for 6 hours to release the oligonucleotide from the solid support. Next, the solvent was removed by concentration, and the free oligonucleotide was dissolved in 3.78 mL of dimethyl sulfoxide. After adding 1.06 mL of acetonitrile, 67 μL of nitromethane, and a stirring bar, 7.02 mL of a 1 M dimethyl sulfoxide solution of tetra-n-butylammonium fluoride (TBAF), dehydrated using molecular sieve 4A, was added dropwise over 1 hour at room temperature under stirring. The mixture was then incubated at 33°C for 4 hours to deprotect 2' PMM protecting groups. The crude product of the oligonucleotide oligomer was then obtained by precipitation.

Example 1

3 mg of the crude product obtained in Reference Example 1 was placed in a 2 mL glass vial (manufactured by Agilent) and dissolved in 1 mL of 50% aqueous acetic acid solution. The vial containing the mixed solution was placed in an incubator (manufactured by Kenis) maintained at 25°C and left to stand for 30 minutes. After standing, the vial was removed from the incubator, and the ratios of FLP and the branched products were calculated using the method described in Measurement Method 1. The results are shown in Table 2.

Example 2

3 mg of the crude product obtained in Reference Example 1 was placed in a 2 mL glass vial (manufactured by Agilent) and dissolved in 1 mL of 10% aqueous acetic acid solution. The vial containing the mixed solution was placed in an incubator (manufactured by Kenis) maintained at 25°C and left to stand for 2 hours. After standing, the vial was removed from the incubator, and the ratios of FLP and the branched products were calculated using the method described in Measurement Method 1. The results are shown in Table 2.

Example 3

3 mg of the crude product obtained in Reference Example 1 was placed in a 2 mL glass vial (manufactured by Agilent) and dissolved in 1 mL of 0.5% aqueous acetic acid solution. The vial containing the mixed solution was placed in an incubator (manufactured by Kenis) maintained at 25°C and left to stand for 24 hours. After standing, the vial was removed from the incubator, and the ratios of FLP and the branched products were calculated using the method described in Measurement Method 1. The results are shown in Table 2.

Example 4

3 mg of the crude product obtained in Reference Example 1 was placed in a 2 mL glass vial (manufactured by Agilent) and dissolved in 1 mL of 0.1M sodium acetate buffer (pH=5.2). The vial containing the mixed solution was placed in an incubator (manufactured by Kenis) maintained at 25°C and left to stand for 48 hours. After standing, the vial was removed from the incubator, and the ratios of FLP and the branched products were calculated using the method described in Measurement Method 1. The results are shown in Table 2.

Example 5

3 mg of the crude product obtained in Reference Example 1 was placed in a 2 mL glass vial (manufactured by Agilent) and dissolved in 1 mL of UF water. The vial containing the mixed solution was placed in an incubator (manufactured by Kenis) maintained at 25°C and left to stand for 1 week. After standing, the vial was removed from the incubator, and the ratios of FLP and the branched products were calculated using the method described in Measurement Method 1. The results are shown in Table 2.

Example 6

3 mg of the crude product obtained in Reference Example 1 was placed in a 2 mL glass vial (manufactured by Agilent) and dissolved in 1 mL of UF water. The vial containing the mixed solution was placed in an incubator (manufactured by Kenis) maintained at 40°C and left to stand for 72 hours. After standing, the vial was removed from the incubator and cooled to room temperature. The ratios of FLP and the branched products were calculated using the method described in Measurement Method 1. The results are shown in Table 2.

Example 7

3 mg of the crude product obtained in Reference Example 1 was placed in a 2 mL glass vial (manufactured by Agilent) and dissolved in 1 mL of UF water. The vial containing the mixed solution was placed in an incubator (manufactured by Kenis) maintained at 50°C and left to stand for 24 hours. After standing, the vial was removed from the incubator and cooled to room temperature. The ratios of FLP and the branched products were calculated using the method described in Measurement Method 1. The results are shown in Table 2.

Example 8

3 mg of the crude product obtained in Reference Example 1 was placed in a 2 mL glass vial (manufactured by Agilent) and dissolved in 1 mL of UF water. The vial containing the mixed solution was placed in an incubator (manufactured by Kenis) maintained at 60°C and left to stand for 6 hours. After standing, the vial was removed from the incubator and cooled to room temperature. The ratios of FLP and the branched products were calculated using the method described in Measurement Method 1. The results are shown in Table 2.

Example 9

3 mg of the crude product obtained in Reference Example 1 was placed in a 2 mL glass vial (manufactured by Agilent) and dissolved in 1 mL of 0.1 M Tris-HCl buffer (pH=7.5). The vial containing the mixed solution was placed in an incubator (manufactured by Kenis) maintained at 60°C and left to stand for 24 hours. After standing, the vial was removed from the incubator and cooled to room temperature. The ratios of FLP and the branched products were calculated using the method described in Measurement Method 1. The results are shown in Table 2.

Comparative Example 1

3 mg of the crude product obtained in Reference Example 1 was placed in a 2 mL glass vial (manufactured by Agilent) and dissolved in 1 mL of 0.1 M Tris-HCl buffer (pH=9.0). The vial containing the mixed solution was placed in an incubator (manufactured by Kenis) maintained at 60°C and left to stand for 6 hours. After standing, the vial was removed from the incubator and cooled to room temperature. The ratios of FLP and the branched products were calculated using the method described in Measurement Method 1. The results are shown in Table 2.

Comparative Example 2 (Initial value: Before reaction)

3 mg of the crude product obtained in Reference Example 1 was dissolved in 1 mL of UF water. The ratios of FLP and the branched products were calculated using the method described in Measurement Method 1. The results are shown in Table 2.

Comparative Example 3 (Reaction conditions described in Non-Patent Literature 2)

Following the method described in Non-Patent Literature 2 (Oligonucleotides 2006, 16, 181-185), the crude oligonucleotide after synthesis was treated using triethylamine trihydrofluoride. Specifically, 3 mg of the crude product obtained in Reference Example 1 was placed in a 2 mL glass vial (manufactured by Agilent) and triethylamine trihydrofluoride was added. The vial containing the mixture was placed in an incubator (manufactured by Kenis) maintained at 65°C and left to stand for 1.5 hours. After standing, the vial was removed from the incubator and cooled to room temperature. The ratios of FLP and the branched products were calculated using the method described in Measurement Method 1. The results are shown in Table 2.

Comparative Example 4 (Reaction conditions described in Non-Patent Literature 3)

Following the method described in Non-Patent Literature 3 (J. Org. Chem., 1970, 35, 3800-3803), the crude oligonucleotide after synthesis was treated at 100°C using 80% aqueous acetic acid solution. Specifically, 3 mg of the crude product obtained in Reference Example 1 was placed in a 2 mL glass vial (manufactured by Agilent) and dissolved in 1 mL of 80% aqueous acetic acid solution. The vial containing the mixed solution was placed in an oil bath maintained at 100°C and left to stand for 20 minutes. After standing, the vial was removed from the oil bath and cooled to room temperature. The ratios of FLP and the branched products were calculated using the method described in Measurement Method 1. The results are shown in Table 2.

The results of Examples 1 to 9 and Comparative Examples 1 to 4 are shown in Table 2. In Table 2, N.D. means not

detected.

[Table 2]

Table 2

| Test Example | Solvent | pH | Reaction Temperature | Reaction Time | Branched Products Ratio (%) | FLP Ratio (%) | Branched Products /FLP (%) |
|---|---|---|---|---|---|---|---|
| Example 1 | 50% acetic acid water | 1.2 | 25°C | 30min | 0.9 | 35.7 | 2.5 |
| Example 2 | 10% acetic acid water | 2.1 | 25°C | 2h | 0.9 | 36.1 | 2.5 |
| Example 3 | 0.5% acetic acid water | 2.9 | 25°C | 24h | 0.8 | 36.5 | 2.2 |
| Example 4 | 0.1M sodium acetate buffer | 5.2 | 25°C | 48h | 1.5 | 33.6 | 4.5 |
| Example 5 | UF water | 6.8 | 25°C | 1week | 1.4 | 34.0 | 4.1 |
| Example 6 | UF water | 6.8 | 40°C | 72h | 1.4 | 34.5 | 4.1 |
| Example 7 | UF water | 6.8 | 50°C | 24h | 1.2 | 35.4 | 3.4 |
| Example 8 | UF water | 6.8 | 60°C | 6h | 1.4 | 34.5 | 4.1 |
| Example 9 | 0.1M Tris-HCl buffer | 7.5 | 60°C | 24h | 1.4 | 31.7 | 4.4 |
| Comparative Example 1 | 0.1M Tris-HCl buffer | 9.0 | 60°C | 6h | 3.9 | 21.6 | 18.1 |
| Comparative Example 2 (Initial value) | UF water | 6.8 | - | - | 5.5 | 25.6 | 21.5 |
| Comparative Example 3 | $Et_3N \cdot 3HF$ | - | 65°C | 1.5h | N. D. | N. D. | - |
| Comparative Example 4 | 80% acetic acid water | Less than 1.0 | 100°C | 20min | N. D. | N. D. | - |

In Table 2, Branched Products Ratio refers to the ratio (area percentage) of branched products in the oligonucleotide, determined by analyzing the oligonucleotide using the aforementioned measurement method 1. Additionally, FLP Ratio refers to the ratio (area percentage) of FLP in the oligonucleotide, determined by analyzing the oligonucleotide using the aforementioned measurement method 1. "Branched Products/FLP" refers to the content ratio of branched products when the ratio of FLP in the oligonucleotide is set to 100% and is calculated using the following formula:

"Branched Products/FLP" (%) = (Branched Products Ratio/FLP Ratio) × 100

INDUSTRIAL APPLICABILITY

By the manufacturing method of the present invention, it is possible to selectively decompose branched products generated in the production of oligonucleotides, thereby improving the yield and purity of the oligonucleotides.

[Free text of Sequence Listing]

Sequence Nos. 1 to 10 in the Sequence Listing represent the base sequences of oligonucleotides that are produced according to the production method of the present invention.

[Sequence Listing]

## Claims

1. A method for producing an oligonucleotide, comprising a step of decomposing branched products by reacting an n-polymerized oligonucleotide (where n is any integer of 2 or more) with water or an aqueous solution having a pH of 1 to 8.

2. The method for producing the oligonucleotide according to claim 1, wherein the step of decomposing the branched products comprises a reaction that selectively cleaves the phosphoramidate bonds of the branched products.

3. The method for producing the oligonucleotide according to claim 1 or 2, wherein a crude oligonucleotide after solid-phase synthesis is used as a starting material.

4. The method for producing the oligonucleotide according to any one of claims 1 to 3, wherein the step of decomposing the branched products comprises a step of reacting for 10 minutes or more by mixing the n-polymerized oligonucleotide (where n is any integer of 2 or more) with the water or the aqueous solution having the pH of 1 to 8.

5. The method according to any one of claims 1 to 4, wherein the reaction temperature is 0 to 60°C.

6. The method according to any one of claims 1 to 5, wherein the water or the aqueous solution having the pH of 1 to 8 is an aqueous solution containing acetic acid or an acetate.

7. The method according to any one of claims 1 to 5, wherein the water or the aqueous solution having the pH of 1 to 8 is a Tris-HCl buffer having a pH of 7 to 8.

8. The method according to any one of claims 1 to 5, wherein the water or the aqueous solution having the pH of 1 to 8 is water.

9. The method according to any one of claims 1 to 8, wherein the n-polymerized oligonucleotide is an n-polymerized oligonucleotide containing a nucleotide having 2'-OMe.

10. The method according to any one of claims 1 to 9, wherein the n-polymerized oligonucleotide is an n-polymerized oligonucleotide containing a nucleotide having 2'-OH.

11. The method according to any one of claims 1 to 10, wherein the pH of the aqueous solution is 1 to 2 and the reaction temperature is 0 to 30°C.

12. The method according to any one of claims 1 to 10, wherein the pH of the aqueous solution is 3 to 4 and the reaction temperature is 0 to 50°C.

13. The method according to any one of claims 1 to 10, wherein the pH of the aqueous solution is 5 to 8 and the reaction temperature is 20 to 60°C.

14. An oligonucleotide wherein the content ratio of the branched products is 15% or less relative to the full-length product (FLP).

15. An oligonucleotide wherein the content ratio of the branched products is 5% or less relative to the FLP.

16. An oligonucleotide with a chain length of 50 or more, wherein the content ratio of the branched products is 5% or less relative to the FLP.

17. An oligonucleotide with a chain length of 100 or more, wherein the content ratio of the branched products is 5% or less relative to the FLP.

18. The method for producing the oligonucleotide according to any one of claims 1 to 13, wherein the oligonucleotide is

RNA.

**19.** The oligonucleotide according to any one of claims 14 to 17, wherein the oligonucleotide is RNA.

[FIG. 1]

Scheme A

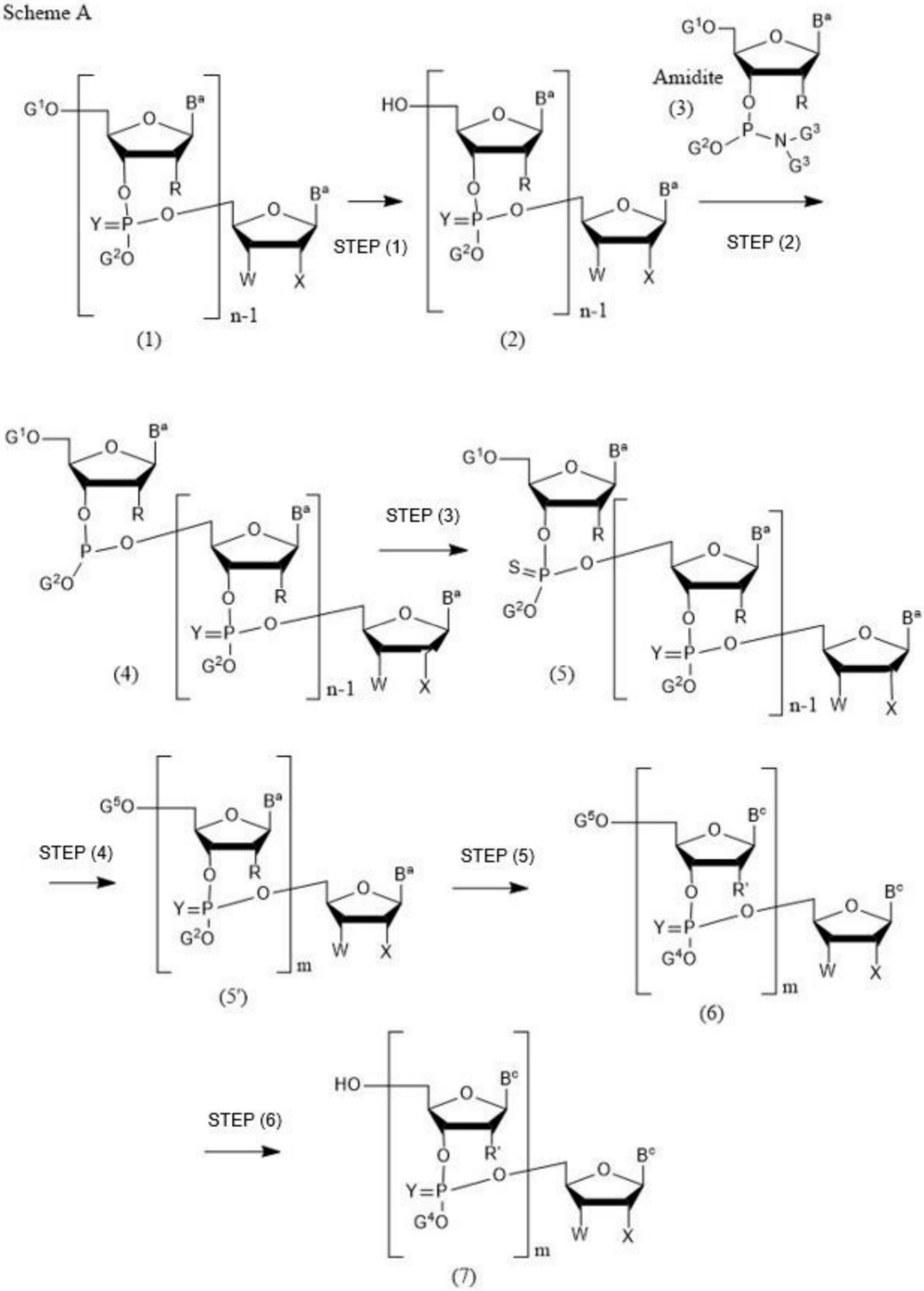

**INTERNATIONAL SEARCH REPORT**

International application No.
**PCT/JP2023/046424**

**A. CLASSIFICATION OF SUBJECT MATTER**

***C07H 21/00***(2006.01)i
FI: C07H21/00 ZNA

According to International Patent Classification (IPC) or to both national classification and IPC

**B. FIELDS SEARCHED**

Minimum documentation searched (classification system followed by classification symbols)

C07H21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2017/104836 A1 (AJINOMOTO CO., INC.) 22 June 2017 (2017-06-22)<br>claims, paragraphs [0253], [0449] | 14-17, 19<br>1-13, 18 |
| Y<br>A | KURATA, C. et al., Characterization of high molecular weight impurities in synthetic phosphorothioate oligonucleotides, Bioorganic & Medicinal Chemistry Letters, 2006, vol. 16, no. 3, pages 607-614, DOI: 10.1016/j.bmcl.2005.10.051<br>Chart I, Oligonucleotide 1, table 1 | 14-17, 19<br>1-13, 18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 March 2024** | **12 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2023/046424**

**C. DOCUMENTS CONSIDERED TO BE RELEVANT**

| Category* | Citation of document, with indication, where appropriate, of the relevant passages | Relevant to claim No. |
|---|---|---|
| Y | 細野宏樹 他, 続・生物工学基礎講座 バイオよもやま話 オリゴヌクレオチド(プライマー)合成, 早さの秘密, 生物工学会誌/日本生物工学会 編, 25 November 2022, vol. 100, no. 11, pages 611-615, DOI: 10.34565/seibutsukogaku.100.11_611, (seibutsu-kogaku kaishi/ edited by the Society for Biotechnology, Japan), non-official translation (HOSONO, Hiroki et al., Continued Basic Bioengineering Course, Bio Yomoyama Story, Oligonucleotide (Primer) Synthesis, Secret of Speed)<br>fig. 1 | 14-17, 19 |
| A | | 1-13, 18 |
| Y | POURSHAHIAN, S., Therapeutic Oligonucleotides, Impurities, Degradants, and Their Characterization by Mass Spectrometry, Mass Spectrometry Reviews, 2019, vol. 40, no. 2, pages 75-109, DOI: 10.1002/mas.21615<br>page 81, upper left column, lines 3-16 | 14-17, 19 |
| A | | 1-13, 18 |
| Y | CAZENAVE, C. et al., Formation of N-Branched Oligonucleotides as By-products in Solid-Phase Oligonucleotide Synthesis, Oligonucleotides, 2006, vol. 16, no. 2, pages 181-185, DOI: 10.1089/oli.2006.16.181<br>page 183, left column, lines 11-14 | 14-17, 19 |
| A | | 1-13, 18 |
| Y | LETSINGER, R. L. et al., Phosphoramidate analogs of oligonucleotides, The Journal of Organic Chemistry, 1970, vol. 35, no. 11, pages 3800-3803, DOI: 10.1021/jo00836a048<br>page 3802, Hydrolytic Degradation | 14-17, 19 |
| A | | 1-13, 18 |
| Y | SEKINE, M. et al., Proton-Block Strategy for the Synthesis of Oligodeoxynucleotides without Base Protection, Capping Reaction, and P-N Bond Cleavage Reaction, The Journal of Organic Chemistry, 2003, vol. 68, no. 14, pages 5478-5492, DOI: 10.1021/jo034204k<br>page 5480, fig. 1 | 14-17, 19 |
| A | | 1-13, 18 |

Form PCT/ISA/210 (second sheet) (July 2022)

**INTERNATIONAL SEARCH REPORT**

International application No.
**PCT/JP2023/046424**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2023/046424**

| Patent document cited in search report | Publication date (day/month/year) | Patent family member(s) | Publication date (day/month/year) |
| --- | --- | --- | --- |
| WO 2017/104836 A1 | 22 June 2017 | US 2018/0282365 A1<br>claims, paragraphs [0563], [0890]<br>EP 3398955 A1<br>CN 108473526 A<br>CN 116063365 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022208672 A
- JP 4705716 B
- JP 5157168 B
- JP 5554881 B
- WO 2006022323 A1
- WO 2013027843 A1
- WO 2019208571 A1
- JP 3745226 B
- WO 2001053528 A1
- JP 2014221817 A
- WO 2019060442 A1
- JP 2015523856 A
- JP 2017537626 A


### Non-patent literature cited in the description


- *Mass Spectrometry, Reviews*, 2021, vol. 40, 75-109
- *Oligonucleotides*, 2006, vol. 16, 181-185
- *J. Org. Chem.*, 1970, vol. 35, 3800-3803
- **XIULONG, SHEN et al.** *Nucleic Acids Research*, 2018, vol. 46 (46), 1584-1600
- **DANIEL O'REILLY et al.** *Nucleic Acids Research*, 2019, vol. 47 (2), 546-558
- *Nucleic Acids Research*, 2019, vol. 47 (2), 547